Europäisches Patentamt

European Patent Office

Office européen des brevets

(19)

(11) EP 0 813 860 B1

# (12) FASCICULE DE BREVET EUROPEEN

(45) Date de publication et mention
de la délivrance du brevet:
**03.02.1999 Bulletin 1999/05**

(51) Int. Cl.⁶: **A61K 7/06**, A61K 7/48,
A61K 7/02, A61K 7/50

(21) Numéro de dépôt: **97401116.5**

(22) Date de dépôt: **21.05.1997**

(54) **Utilisation de dérivés de l'anhydride succinique dans des compositions de nettoyage de la peau**

Verwendung von Bernsteinsäureanhydrid-Derivaten in Zusammensetzungen zur Reinigung der Haut

Use of derivatives of succinic anhydrides in skin cleansing compositions

(84) Etats contractants désignés:
**DE ES FR GB IT**

(30) Priorité: **21.06.1996 FR 9607777**

(43) Date de publication de la demande:
**29.12.1997 Bulletin 1997/52**

(73) Titulaire: **L'OREAL**
**75008 Paris (FR)**

(72) Inventeurs:
• **Simon, Pascal**
**94400 Vitry Sur Seine (FR)**

• **Bollens, Eric**
**94130 Nogent Sur Marne (FR)**
• **Gagnebien, Didier**
**Westfield 07090 New Jersey (US)**

(74) Mandataire: **Dodin, Catherine**
**L'Oreal-D.P.I.,**
**90, rue du Général Roguet**
**92583 Clichy Cédex (FR)**

(56) Documents cités:
EP-A- 0 210 642          WO-A-94/11333
WO-A-95/35088          DE-A-19 505 100

**Description**

L'invention a pour objet l'utilisation comme tensioactifs de produits dérivant de la condensation de dérivés d'anhydride alkyle ou alcényl succinique avec des polyoxyalkylènes, pour le démaquillage et le nettoyage de la peau, des cheveux et des muqueuses.

L'utilisateur attend d'une composition démaquillante qu'elle permette d'ôter toutes sortes de produits de maquillage: rouge à lèvres, poudre, ombre à paupières, fond de teint etc., sans abîmer la peau et en la laissant propre et douce.

Parmi les produits de démaquillage, beaucoup ont des bases aqueuses: lotions aqueuses, hydroalcooliques, hydroglycoliques, gels aqueux transparents. Ils contiennent des tensioactifs solubilisés qui assurent le démaquillage par la mise en suspension des produits de maquillage. Ces tensioactifs doivent avoir une bonne efficacité de démaquillage et une bonne tolérance et doivent assurer une bonne stabilité à la formule.

Le plus souvent, un tensioactif efficace, comme par exemple un tensioactif de la classe des anioniques, est mal toléré. Au contraire un tensioactif doux, comme par exemple un tensioactif non-ionique, a souvent une activité détergente insuffisante. On peut rechercher une meilleure efficacité de démaquillage en augmentant les quantités de tensioactif dans la composition, mais on risque alors de se heurter à des problèmes de stabilité : recristallisation, coloration, mauvaise tenue à la lumière et à la température. Il subsiste donc le besoin de disposer d'un tensioactif peu irritant donc non-ionique qui possède toutefois de bonnes propriétés de démaquillage à faible concentration.

On connaît par le document WO-94/00508 les propriétés émulsionnantes et dispersantes de produits issus de la condensation de dérivés alkyle succiniques sur des dérivés polyoxyalkylénés. Ces tensioactifs non-ioniques sont réputés avoir une très bonne biodégradabilité.

Par ailleurs, on connaît par les documents WO-95/35088 et WO-94/10971 l'utilisation, dans le soin de la peau, de produits issus de la condensation de dérivés alkyle ou alcényl succiniques sur des dérivés polyoxyéthylénés. Les produits décrits dans ces documents ont des propriétés dans le soin de la peau qui les rendent comparables aux céramides (traitement des peaux sèches abîmées ou âgées, amélioration de la flexibilité du Stratum Corneum, rétention de l'eau par la peau) et sont en outre plus solubles que ces derniers dans des compositions cosmétiques.

Toutefois, rien ne laissait supposer jusqu'à présent que les composés décrits dans ces documents pouvaient posséder une quelconque propriété nettoyante et/ou démaquillante de la peau, des muqueuses, des semi-muqueuses et/ou des cheveux.

Aussi c'est avec étonnement que la demanderesse a découvert que certains tensioactifs issus de la condensation de dérivés d'anhydride alkyle ou alcényl succinique avec des polyoxyalkylènes pouvaient être utilisés avec succès dans des compositions cosmétiques pour le démaquillage de la peau et le nettoyage de la peau et des cheveux. Ces tensioactifs possèdent une bonne efficacité de démaquillage et de nettoyage, alliées à une grande douceur et permettent d'obtenir des formules qui sont durablement stables.

De plus, ils peuvent être utilisés seuls ou en combinaison avec d'autres tensioactifs usuels.

L'invention a pour objet l'utilisation dans des compositions cosmétiques pour le nettoyage et/ou le démaquillage de la peau, des muqueuses, des semi-muqueuses et/ou des phanères telles que les cheveux, d'au moins un dérivé de l'anhydride succinique répondant à l'une des formules I et II :

$$GOOC\text{-}(HR)C\text{-}C(HR')\text{-}COO\text{-}(C_mH_{2m}O)_n\text{-}H \tag{I}$$

$$GOOC\text{-}(HR)C\text{-}C(HR')\text{-}COO\text{-}(C_mH_{2m}O)_n\text{-}C_pH_{2p}\text{-}O\text{-}CO\text{-}(HR')C\text{-}C(HR)\text{-}COOG \tag{II}$$

dans lesquelles :

- l'un des radicaux R et R' est choisi parmi les radicaux alkyle et alcényle en $C_6\text{-}C_{22}$, linéaires ou ramifiés, et l'autre radical est l'atome d'hydrogène,
- n va de 2 à 100,
- m est égal à 2 ou 3, et peut varier le long de la chaîne polyoxyalkylène,
- p est égal à 2 ou 3,
- G est choisi parmi l'atome d'hydrogène ; un groupement alkyle en $C_1\text{-}C_6$ ; un radical $-(C_{m'}H_{2m'}O)_{n'}H$, dans lequel n' va de 2 à 100 et m' est égal à 2 ou 3 et peut varier le long de la chaîne ; un cation,

avec la réserve que lorsque G est un radical $-(C_{m'}H_{2m'}O)_{n'}H$, alors l'un au moins de m et de m'est égal à 2.

Dans les formules (I) et (II), n et n' sont des valeurs moyennes et ne sont donc pas forcément des entiers. On choisit avantageusement pour n une valeur allant de 5 à 60 et encore plus préférentiellement de 10 à 30.

Les composés utilisés selon l'invention comprennent au moins une chaîne polyoxyalkylène composée de résidus oxyéthylène et/ou oxypropylène. La chaîne peut être une chaîne homopolymérique de résidus éthylène glycol ou de résidus propylène glycol ou elle peut être un copolymère bloc ou aléatoire contenant à la fois des résidus éthylène glycol et propylène glycol. Parmi les composés comprenant plus d'une chaîne polyoxyalkylène, les chaînes peuvent être identiques ou différentes.

Lorsque G représente un radical -$(C_{m'}H_{2m'}O)_{n'}H$, on a de préférence : n = n' et m = m'.

Lorsque G est un cation, il peut être choisi parmi un ion d'une base minérale ou organique, comme par exemple un ion d'un métal alcalin, parmi lesquels on peut citer $Na^+$ ou $K^+$ ou l'ion ammonium quaternaire, un sel basique de lysine ou d'arginine.

Préférentiellement G représente l'atome d'hydrogène.

Avantageusement on choisit le radical R ou R' qui est distinct de H parmi les radicaux alkyl 1,2-ène linéaires en $C_8$-$C_{22}$.

Les composés de formules (I) et (II) décrits ci-dessus peuvent être préparés conformément à la description qui est donnée dans la demande de brevet publiée sous le numéro WO-94/00508, ou dans l'un des brevets GB-2131820 et EP-0107199.

Un autre objet de l'invention est l'utilisation dans une composition cosmétique ou hygiénique, en tant qu'agent nettoyant et/ou démaquillant de la peau, des muqueuses, des semi-muqueuses et/ou des phanères telles que les cheveux, d'au moins un dérivé de l'anhydride succinique répondant à l'une des formules (I) et (II) ci-dessus.

Un autre objet de l'invention est un procédé de nettoyage et/ou de démaquillage d'un support tel que de la peau, les muqueuses, les semi-muqueuses, les phanères et/ou les yeux, caractérisé en ce qu'on applique sur ledit support une composition comprenant au moins un dérivé de l'anhydride succinique répondant à l'une des formules (I) et (II).

Ladite composition peut se présenter sous la forme d'une émulsion, notamment huile-dans-eau ou eau-dans-huile, voire sous la forme d'une émulsion multiple.

Elle peut également se présenter sous forme d'une solution aqueuse, éventuellement gélifiée, ou sous forme d'une lotion, par exemple biphasée, d'une lotion aqueuse, ou hydroalcoolique, d'une crème, d'un lait, voire d'une mousse.

La composition utilisée selon l'invention peut comprendre une phase grasse à base d'huile animale, végétale, minérale, siliconée, fluorée et/ou synthétique. La phase grasse peut également comprendre des alcools gras ou des acides gras, ainsi que des tensioactifs.

On peut notamment citer les huiles hydrocarbonées telles que l'huile de paraffine ou de vaseline; le perhydrosqualène; l'huile d'Arara; l'huile d'amande douce, de calophyllum, de palme, de ricin, d'avocat, de jojoba, d'olive ou de germes de céréales; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones.

La phase grasse peut également comprendre une huile démaquillante telle qu'un ester d'acides gras, en particulier les esters obtenus à partir d'un alcool à chaîne droite ou ramifiée ayant de 1 à 17 atomes de carbone et d'un acide gras à chaîne droite ou ramifiée ayant de 3 à 18 atomes de carbone.

Un tel ester peut en particulier être choisi dans le groupe constitué par l'adipate de dioctyle, le palmitate d'éthyl-2 hexyle, l'adipate de diisopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.

La phase grasse peut être présente à raison de 5-95% en poids dans le cas d'une émulsion.

La composition utilisée selon l'invention peut comprendre, en outre,

- un agent permettant la mise en suspension de la phase grasse, par exemple un copolymère d'acrylates d'alkyle en $C_{10}$-$C_{30}$ et d'acide acrylique ou méthacrylique ou de leur ester (Pemulen TR1$^R$, Pemulen TR2$^R$, Carbopol 1342$^R$ de GOODRICH); ou un copolymère acrylamide/acide méthylpropane sulfonique (Sepigel$^R$ de SEPPIC), et/ou
- un agent de mise en dispersion de la phase grasse, tel qu'un système émulsionnant ou vésiculaire à base de vésicules, éventuellement de taille nanométrique, constituées de lipides ioniques (liposomes) ou non ioniques, et en particulier les systèmes émulsionnants bien connus de l'homme du métier constitués de stéarate de glycéryl /PEG 100 stéarate (CTFA), d'alcool cétylique et d'alcool stéarylique.

La composition utilisée selon l'invention peut comprendre, en outre, un agent pour modifier sa viscosité, et obtenir des textures plus ou moins gélifiées, tel que :

- les dérivés cellulosiques (carboxyméthylcellulose, hydroxyéthylcellulose, hydroxypropylméthylcellulose),
- les gommes naturelles telles que les gommes de xanthane, de guar, de caroube, les scléroglucanes, les dérivés de chitine ou de chitosane, les carraghénanes,
- les dérivés polycarboxyvinyliques du type Carbomer (vendues par GOODRICH sous les dénominations Carbopol 940$^R$, 951$^R$, 980$^R$, ou par 3V-SIGMA sous la dénomination Synthalen K$^R$ ou Synthalen L$^R$).

La composition utilisée selon l'invention peut également comprendre, de façon connue, des adjuvants couramment utilisés dans le domaine considéré, tels que les conservateurs, les antioxydants, les parfums, les charges telles que le kaolin ou l'amidon, voire les microsphères creuses, les pigments, les filtres UV, les séquestrants, les huiles essentielles, les matières colorantes, les actifs hydrophiles ou lipophiles tels que les hydratants, notamment la glycérine, le butylène glycol, les anti-inflammatoires comme l'allantoïne, le bisabolol, les anti-radicaux libres comme la vitamine E ou ses dérivés, les apaisants tels que l'eau de bleuet, l'extrait d'iris, les dépigmentants, les actifs biologiques tels que l'urée, les acides aminés, les vitamines et leurs dérivés, les protéines, l'acide salicylique et ses dérivés, les α-hydroxyacides, l'acide pyrrolidone carboxylique et ses sels, les céramides.
Bien entendu l'homme du métier veillera à choisir ce ou ces éventuels composés complémentaires, et/ou leur quantité, de manière telle que les propriétés avantageuses de la composition utilisée selon l'invention ne soient pas, ou substantiellement pas, altérées par l'adjonction envisagée.

Préférentiellement, la composition utilisée selon l'invention se présente sous la forme d'une solution aqueuse, éventuellement gélifiée, d'une lotion biphasée, d'une lotion aqueuse simple, ou hydroalcoolique : ces formes ne nécessitant pas, pour assurer leur stabilité, l'emploi d'autres tensioactifs, elles présentent une meilleure tolérance par tous les types de peaux, en particulier les peaux sensibles.
La composition présente de préférence un pH qui respecte la peau, généralement compris entre 5 et 8, de préférence un pH de 5,5 à 7,5.

Les compositions utilisées selon l'invention trouvent en particulier une application en tant que lait ou lotion démaquillante pour les yeux et/ou la peau, notamment la peau du visage, ainsi qu'en tant que shampooing et/ou gel douche.

Des exemples de formules sont donnés ci-dessous, qui permettent d'illustrer l'invention sans limiter sa portée. Dans tous ces exemples, les pourcentages sont donnés en poids.
Les quantités sont évaluées en matière active.

Les composés E1, E2 et E3 utilisés dans ces exemples sont préparés conformément aux exemples 1 à 3 du brevet EP-0107199 (on peut également se référer à la demande de brevet WO-94/00508 ou au brevet GB-2131820) :

    E1 : formule (I) avec R= n-dodécyl-trans-2-ène, R'=H, m=2, n=18, G=H
    E2 : formule (I) avec R= n-hexadécyl-trans-2-ène, R'=H, m=2, n=18, G=H
    E3 : formule (II) avec R= n-hexadécyl-trans-2-ène, R'=H, m=2, n=18, p= G=H

<u>Exemple 1</u>

On prépare des laits démaquillants sous la forme d'une émulsion H/E ayant la formule suivante :

| | |
|---|---|
| Palmitate d'éthyl-2-hexyle | 15% |
| Composé Ei (i=1,2,3) | 1,5% |
| Pemulen TR2$^R$(GOODRICH) | 0,7% |
| Triéthanolamine | 0,5% |
| Conservateur | 0,2% |
| Parfum | 0,3% |
| Eau | qsp 100 |

Ces laits donnent un très bon démaquillage, sans picotement autour des yeux. Ils laissent la peau douce et propre.

Exemple 2

On prépare des lotions démaquillantes de formule :

| | |
|---|---|
| Composé Ei (i=1,2,3) | 0,8% |
| Poloxamer 184 * | 1% |
| Conservateur | 0,15% |
| Parfum | 0,2% |
| Eau | qsp 100 |

*nom CTFA d'un polymère bloc polyoxyde d'éthylène-polyoxyde de propylène.

Ces lotions donnent un bon résultat de démaquillage, sans picotements ni irritations. Elles laissent sur la peau une impression de fraîcheur.

Exemple 3

On prépare des compositions utilisées conformément à l'invention :

Composition $C_i$ :

| | |
|---|---|
| Paraben | 0,2% |
| EDTA | 0,1% |
| Composé Ei (i=1, 2, 3) | 0,65% |
| Eau | qsp100 |

On utilise comme témoin une composition ayant le même pourcentage de tensioactif que les compositions utilisées selon l'invention Ci (i=1,2,3), cette composition-témoin étant connue pour ses bonnes propriétés démaquillantes et comprenant :

Composition T :

| | |
|---|---|
| Paraben | 0,2% |
| EDTA | 0,1% |
| Lauryl éther sulfate de sodium | 0,23% |
| Coco-amphodiacétate | 0,42% |
| Eau | qsp100 |

A) Pouvoir démaquillant ⟨⟨in vitro⟩⟩ :

Le pouvoir démaquillant de ces compositions est contrôlé à l'aide de la technique dite du "robot démaquilleur" :

. l'appareil se compose d'une plaque et d'un bras muni d'un poids exerçant sur la plaque une pression de 100 g/cm$^2$ et équipé à l'une de ses extrémités d'un coton qui glisse sur la plaque.
. on dépose sur la plaque une fine couche d'un mascara waterproof noir commercialisé sous le nom d'Aquacils par la société Lancôme.
. cette plaque est mise à sécher pendant 4 h.
. le coton est imbibé de quarante gouttes de la composition démaquillante à tester et on réalise un aller-retour sur la plaque contenant le mascara.
. cette opération est renouvelée tant que la plaque n'est pas exempte de mascara, le coton étant changé après chaque passage.
. on note le nombre de cotons nécessaire à un démaquillage parfait.

L'absence totale de mascara sur la plaque constitue, selon ce test, un démaquillage parfait.
Il faut deux aller et retour pour ôter la totalité du mascara de la plaque du robot-démaquilleur avec l'une quelconque des compositions Ci, de même qu'avec la composition témoin T.

B) Pouvoir démaquillant ⟨⟨in vitro⟩⟩ :

On fait des essais de démaquillage des yeux sur des femmes, par demi-visage : une moitié du visage est démaquillée avec la composition C2 et l'autre moitié avec la composition témoin T.
On effectue le test sur 11 personnes.
On note leurs observations : le pouvoir démaquillant de la composition C2 est supérieur pour 60% des personnes.
Ces deux tests montrent que les compositions démaquillantes utilisées selon l'invention sont d'une grande efficacité.
Elles évitent l'emploi de tensioactifs amphotères et anioniques, plus irritants.

C) Tolérance oculaire :

La tolérance oculaire est mesurée par la détermination du paramètre LSR (Lacrymal Surface Response).
Les compositions de tensioactif sont diluées par un liquide lacrymal de synthèse contenant une protéine modèle : l'albumine de sérum bovin. On enregistre les courbes de variation de la tension superficielle statique des solutions en fonction de la concentration du tensioactif dans ces solutions à la température de 25°C à l'aide d'un tensiomètre à anneau Krüss K12 équipé d'une burette automatique. La solution mère a une concentration de 1 ou 2 g/l, la concentration initiale des mesures est de 20 ou 50 mg/l, la concentration finale est de 600 ou 1200 g/l, le nombre de points pris en compte est de 33.
Le paramètre LSR est calculé à partir de la dérivée de la courbe g = f(log[C]) dans laquelle g représente la tension superficielle et [C] représente la concentration du tensioactif dans la solution.
Ce paramètre est calculé suivant la formule : LSR=[d.dg/dd]d=d$_0$ dans laquelle d représente le facteur de dilution.
Le paramètre LSR de la solution témoin T est de 24, celui de la composition C2 est inférieur à 6, ce qui indique une tolérance oculaire très supérieure pour cette composition par rapport à la composition témoin.

**Revendications**

1. Utilisation dans des compositions cosmétiques pour le nettoyage et/ou le démaquillage de la peau, des muqueuses, des semi-muqueuses et/ou des phanères telles que les cheveux, d'au moins un dérivé de l'anhydride succinique répondant à l'une des formules I et II :

$$GOOC-(HR)C-C(HR')-COO-(C_mH_{2m}O)_n-H \tag{I}$$

$$GOOC-(HR)C-C(HR')-COO-(C_mH_{2m}O)_n-C_pH_{2p}-O-CO-(HR')C-C(HR)-COOG \tag{II}$$

dans lesquelles :

- l'un des radicaux R et R' est choisi parmi les radicaux alkyle et alcényle en $C_6$-$C_{22}$, linéaires ou ramifiés, et l'autre radical est l'atome d'hydrogène,

- n va de 2 à 100,
- m est égal à 2 ou 3, et peut varier le long de la chaîne polyoxyalkylène,
- p est égal à 2 ou 3,
- G est choisi parmi l'atome d'hydrogène; un groupement alkyle en $C_1$-$C_6$ ; un radical -$(C_{m'}H_{2m'}O)_{n'}$H, dans lequel n' va de 2 à 100 et m' est égal à 2 ou 3 et peut varier le long de la chaîne ; un cation,

avec la réserve que lorsque G est un radical -$(C_{m'}H_{2m'}O)_{n'}$H, alors l'un au moins de m et de m'est égal à 2.

2. Utilisation dans une composition cosmétique ou hygiénique, en tant qu'agent nettoyant et/ou démaquillant de la peau, des muqueuses, des semi-muqueuses et/ou des phanères telles que les cheveux, d'au moins un dérivé de l'anhydride succinique répondant à l'une des formules I et II :

$$GOOC\text{-}(HR)C\text{-}C(HR')\text{-}COO\text{-}(C_mH_{2m}O)_n\text{-}H \qquad (I)$$

$$GOOC\text{-}(HR)C\text{-}C(HR')\text{-}COO\text{-}(C_mH_{2m}O)_n\text{-}C_pH_{2p}\text{-}O\text{-}CO\text{-}(HR')C\text{-}C(HR)\text{-}COOG \qquad (II)$$

dans lesquelles :

- l'un des radicaux R et R' est choisi parmi les radicaux alkyle et alcényle en $C_6$-$C_{22}$, linéaires ou ramifiés, et l'autre radical est l'atome d'hydrogène,
- n va de 2 à 100,
- m est égal à 2 ou 3, et peut varier le long de la chaîne polyoxyalkylène,
- p est égal à 2 ou 3,
- G est choisi parmi l'atome d'hydrogène ; un groupement alkyle en $C_1$-$C_6$ ; un radical -$(C_{m'}H_{2m'}O)_{n'}$H, dans lequel n' va de 2 à 100 et m' est égal à 2 ou 3 et peut varier le long de la chaîne ; un cation,

avec la réserve que lorsque G est un radical -$(C_{m'}H_{2m'}O)_{n'}$H, alors l'un au moins de m et de m'est égal à 2.

3. Utilisation selon l'une quelconque des revendications précédentes, dans laquelle G représente un radical -$(C_{m'}H_{2m'}O)_{n'}$H, et n = n' et m = m'.

4. Utilisation selon l'une quelconque des revendications 1 à 2, dans laquelle G est choisi parmi un ion d'une base minérale ou organique, tel qu'un ion d'un métal alcalin, notamment $Na^+$ ou $K^+$ ou l'ion ammonium quaternaire, un sel basique de lysine ou d'arginine.

5. Utilisation selon l'une quelconque des revendications 1 à 2, dans laquelle G représente l'atome d'hydrogène.

6. Utilisation selon l'une quelconque des revendications précédentes, dans laquelle n va de 5 à 60, de préférence de 10 à 30.

7. Utilisation selon l'une quelconque des revendications précédentes, dans laquelle le radical R ou R', distinct de H, est choisi parmi les radicaux alkyl 1,2-ène linéaires en $C_8$-$C_{22}$.

8. Utilisation selon l'une quelconque des revendications 1 à 2, dans laquelle le dérivé de l'anhydride succinique est choisi parmi les composés répondant à l'une des formules suivantes :

$HOOC\text{-}(HR)C\text{-}CH_2\text{-}COO\text{-}(C_2H_4O)_{18}\text{-}H$ avec R= n-dodécyl-trans-2-ène,
$HOOC\text{-}(HR)C\text{-}CH_2\text{-}COO\text{-}(C_2H_4O)_{18}\text{-}H$ avec R= n-hexadécyl-trans-2-ène, et
$HOOC\text{-}(HR)C\text{-}CH_2\text{-}COO\text{-}(C_2H_4O)_{18}\text{-}C_pH_{2p}\text{-}O\text{-}CO\text{-}CH_2\text{-}C(HR)\text{-}COOH$ avec R= n-hexadécyl-trans-2-ène.

9. Utilisation selon l'une quelconque des revendications précédentes, dans laquelle la composition est sous la forme d'une émulsion huile-dans-eau ou eau-dans-huile, d'une émulsion multiple, d'une solution aqueuse, d'une solution aqueuse gélifiée, sous forme d'une lotion, d'une lotion biphasée, d'une lotion aqueuse, d'une lotion hydroalcoolique, d'une crème, d'un lait ou d'une mousse.

10. Utilisation selon l'une quelconque des revendications précédentes, dans laquelle la composition comprend en outre au moins un tensioactif et/ou au moins une huile démaquillante.

**11.** Utilisation selon la revendication 10, dans laquelle l'huile démaquillante est choisie parmi les esters obtenus à partir d'un alcool à chaîne droite ou ramifiée ayant de 1 à 17 atomes de carbone et d'un acide gras à chaîne droite ou ramifiée ayant de 3 à 18 atomes de carbone.

**12.** Utilisation selon l'une quelconque des revendications précédentes, dans laquelle la composition présente un pH compris entre 5 et 8, de préférence entre 5,5 et 7,5.

**13.** Utilisation selon l'une quelconque des revendications précédentes, dans laquelle la composition est un lait ou une lotion démaquillante pour les yeux et/ou la peau, notamment la peau du visage, un shampooing et/ou un gel douche.

**14.** Procédé de nettoyage et/ou de démaquillage d'un support tel que de la peau, les muqueuses, les semi-muqueuses, les phanères et/ou les yeux, caractérisé en ce qu'on applique sur ledit support une composition comprenant au moins un dérivé de l'anhydride succinique répondant à l'une des formules I et II :

$$GOOC\text{-}(HR)C\text{-}C(HR')\text{-}COO\text{-}(C_mH_{2m}O)_n\text{-}H \qquad (I)$$

$$GOOC\text{-}(HR)C\text{-}C(HR')\text{-}COO\text{-}(C_mH_{2m}O)_n\text{-}C_pH_{2p}\text{-}O\text{-}CO\text{-}(HR')C\text{-}C(HR)\text{-}COOG \qquad (II)$$

dans lesquelles :

- l'un des radicaux R et R' est choisi parmi les radicaux alkyle et alcényle en $C_6$-$C_{22}$, linéaires ou ramifiés, et l'autre radical est l'atome d'hydrogène,
- n va de 2 à 100,
- m est égal à 2 ou 3, et peut varier le long de la chaîne polyoxyalkylène,
- p est égal à 2 ou 3,
- G est choisi parmi l'atome d'hydrogène; un groupement alkyle en $C_1$-$C_6$ ; un radical -$(C_{m'}H_{2m'}O)_{n'}$H, dans lequel n' va de 2 à 100 et m' est égal à 2 ou 3 et peut varier le long de la chaîne ; un cation,

avec la réserve que lorsque G est un radical -$(C_{m'}H_{2m'}O)_{n'}$H, alors l'un au moins de m et de m' est égal à 2.

## Claims

**1.** Use, in cosmetic compositions for cleansing and/or removing make-up from the skin, mucous membranes, semi-mucous membranes and/or the exoskeleton such as the hair, of at least one succinic anhydride derivative corresponding to one of the formulae I and II:

$$GOOC\text{-}(HR)C\text{-}C(HR')\text{-}COO\text{-}(C_mH_{2m}O)_n\text{-}H \qquad (I)$$

$$GOOC\text{-}(HR)C\text{-}C(HR')\text{-}COO\text{-}(C_mH_{2m}O)_n\text{-}C_pH_{2p}\text{-}O\text{-}CO\text{-}(HR')C\text{-}C(HR)\text{-}COOG \qquad (II)$$

in which:

- one of the radicals R and R' is chosen from linear or branched $C_6$-$C_{22}$ alkyl and alkenyl radicals and the other radical is a hydrogen atom,
- n ranges from 2 to 100,
- m is equal to 2 or 3 and may vary along the polyoxyalkylene chain,
- p is equal to 2 or 3,
- G is chosen from a hydrogen atom; a $C_1$-$C_6$ alkyl group; a radical -$(C_{m'}H_{2m'}O)_{n'}$H, in which n' ranges from 2 to 100 and m' is equal to 2 or 3 and may vary along the chain; a cation,

with the proviso that, when G is a radical -$(C_{m'}H_{2m'}O)_{n'}$H, then at least one of m and of m' is equal to 2.

**2.** Use, in a cosmetic or hygiene composition, as an agent for cleansing and/or removing make-up from the skin, mucous membranes, semi-mucous membranes and/or the exoskeleton such as the hair, of at least one succinic anhydride derivative corresponding to one of the formulae I and II:

$$GOOC\text{-}(HR)C\text{-}C(HR')\text{-}COO\text{-}(C_mH_{2m}O)_n\text{-}H \qquad (I)$$

$$\text{GOOC-(HR)C-C(HR')-COO-}(C_mH_{2m}O)_n\text{-}C_pH_{2p}\text{-O-CO-(HR')C-C(HR)-COOG} \qquad \text{(II)}$$

in which:

- one of the radicals R and R' is chosen from linear or branched $C_6$-$C_{22}$ alkyl and alkenyl radicals and the other radical is a hydrogen atom,
- n ranges from 2 to 100,
- m is equal to 2 or 3 and may vary along the polyoxyalkylene chain,
- p is equal to 2 or 3,
- G is chosen from a hydrogen atom; a $C_1$-$C_6$ alkyl group; a radical $-(C_{m'}H_{2m'}O)_{n'}H$, in which n' ranges from 2 to 100 and m' is equal to 2 or 3 and may vary along the chain; a cation,

with the proviso that, when G is a radical $-(C_{m'}H_{2m'}O)_{n'}H$, then at least one of m and of m' is equal to 2.

3. Use according to any one of the preceding claims, in which G represents a radical $-(C_{m'}H_{2m'}O)_{n'}H$, and n = n' and m = m'.

4. Use according to either of Claims 1 and 2, in which G is chosen from an ion of an inorganic or organic base, such as an ion of an alkali metal, in particular $Na^+$ or $K^+$, or the quaternary ammonium ion, or a basic salt of lysine or of arginine.

5. Use according to either of Claims 1 and 2, in which G represents a hydrogen atom.

6. Use according to any one of the preceding claims, in which n ranges from 5 to 60, preferably from 10 to 30.

7. Use according to any one of the preceding claims, in which the radical R or R' which is other than H is chosen from linear $C_8$-$C_{22}$ 1,2-alkylene radicals.

8. Use according to either of Claims 1 and 2, in which the succinic anhydride derivative is chosen from compounds corresponding to one of the following formulae:

$HOOC\text{-}(HR)C\text{-}CH_2\text{-}COO\text{-}(C_2H_4O)_{18}\text{-}H$ with R= trans-2-n-dodecylene,
$HOOC\text{-}(HR)C\text{-}CH_2\text{-}COO\text{-}(C_2H_4O)_{18}\text{-}H$ with R= trans-2-n-hexadecylene, and
$HOOC\text{-}(HR)C\text{-}CH_2\text{-}COO\text{-}(C_2H_4O)_{18}\text{-}C_pH_{2p}\text{-}O\text{-}CO\text{-}CH_2\text{-}C(HR)\text{-}COOH$ with R= trans-2-n-hexadecylene.

9. Use according to any one of the preceding claims, in which the composition is in the form of an oil-in-water or water-in-oil emulsion, a multiple emulsion, an aqueous solution, a gelled aqueous solution, in the form of a lotion, a two-phase lotion, an aqueous lotion, an aqueous-alcoholic lotion, a cream, a milk or a foam.

10. Use according to any one of the preceding claims, in which the composition also comprises at least one surfactant and/or at least one make-up-removing oil.

11. Use according to Claim 10, in which the make-up-removing oil is chosen from esters obtained from a straight- or branched-chain alcohol having from 1 to 17 carbon atoms and from a straight- or branched-chain fatty acid having from 3 to 18 carbon atoms.

12. Use according to any one of the preceding claims, in which the composition has a pH of between 5 and 8, preferably of between 5.5 and 7.5.

13. Use according to any one of the preceding claims, in which the composition is a make-up-removing milk or lotion for the eyes and/or the skin, in particular facial skin, a shampoo and/or a shower gel.

14. Process for cleansing and/or removing make-up from a support such as the skin, mucous membranes, semi-mucous membranes, the exoskeleton and/or the eyes, characterized in that a composition is applied to the said support, this composition comprising at least one succinic anhydride derivative corresponding to one of the formulae I and II:

$$\text{GOOC-(HR)C-C(HR')-COO-}(C_mH_{2m}O)_n\text{-H} \qquad \text{(I)}$$

$$GOOC\text{-}(HR)C\text{-}C(HR')\text{-}COO\text{-}(C_mH_{2m}O)_n\text{-}C_pH_{2p}\text{-}O\text{-}CO\text{-}(HR')C\text{-}C(HR)\text{-}COOG \qquad (II)$$

in which:

- one of the radicals R and R' is chosen from linear or branched $C_6$-$C_{22}$ alkyl and alkenyl radicals and the other radical is a hydrogen atom,
- n ranges from 2 to 100,
- m is equal to 2 or 3 and may vary along the polyoxyalkylene chain,
- p is equal to 2 or 3,
- G is chosen from a hydrogen atom; a $C_1$-$C_6$ alkyl group; a radical -$(C_{m'}H_{2m'}O)_{n'}H$, in which n' ranges from 2 to 100 and m' is equal to 2 or 3 and may vary along the chain; a cation,

with the proviso that, when G is a radical -$(C_{m'}H_{2m'}O)_{n'}H$, then at least one of m and of m' is equal to 2.

## Patentansprüche

1. Verwendung mindestens eines Bernsteinsäureanhydrid-Derivats gemäß einer der folgenden Formeln I und II in kosmetischen Zusammensetzungen zur Reinigung und/oder zum Abschminken der Haut, der Schleimhäute, der Semi-Schleimhäute und/oder von Keratinsubstanzen, wie dem Haar:

$$GOOC\text{-}(HR)C\text{-}C(HR')\text{-}COO\text{-}(C_mH_{2m}O)_n\text{-}H \qquad (I)$$

$$GOOC\text{-}(HR)C\text{-}C(HR')\text{-}COO\text{-}(C_mH_{2m}O)_n\text{-}C_pH_{2p}\text{-}O\text{-}CO\text{-}(HR')C\text{-}C(HR)\text{-}COOG \qquad (II)$$

worin

- eine der Gruppen R und R' unter den geradkettigen oder verzweigten Alkyl- und Alkenylgruppen mit 6 bis 22 Kohlenstoffatomen ausgewählt ist und die andere Gruppe Wasserstoff bedeutet,
- n im Bereich von 2 bis 100 liegt,
- m 2 oder 3 bedeutet und daher die Länge der Polyalkylenoxidkette variieren kann,
- p 2 oder 3 bedeutet und
- G ausgewählt ist unter: Wasserstoff; einer $C_{1-6}$-Alkylgruppe, einer Gruppe -$(C_{m'}H_{2m'}O)_{n'}H$, worin n' im Bereich von 2 bis 100 liegt und m' 2 oder 3 bedeutet und daher die Länge der Kette variieren kann; und einem Kation,

mit der Maßgabe, daß mindestens einer der Parameter m und m' 2 bedeutet, wenn G eine Gruppe -$(C_{m'}H_{2m'}O)_{n'}H$ ist.

2. Verwendung mindestens eines Bernsteinsäureanhydrid-Derivats gemäß einer der folgenden Formeln I und II als Mittel zur Reinigung und/oder zum Abschminken der Haut, der Schleimhäute, der Semi-Schleimhäute und/oder von Keratinsubstanzen wie dem Haar:

$$GOOC\text{-}(HR)C\text{-}C(HR')\text{-}COO\text{-}(C_mH_{2m}O)_n\text{-}H \qquad (I)$$

$$GOOC\text{-}(HR)C\text{-}C(HR')\text{-}COO\text{-}(C_mH_{2m}O)_n\text{-}C_pH_{2p}\text{-}O\text{-}CO\text{-}(HR')C\text{-}C(HR)\text{-}COOG \qquad (II),$$

worin

- eine der Gruppen R und R' unter den geradkettigen oder verzweigten Alkyl- und Alkenylgruppen mit 6 bis 22 Kohlenstoffatomen ausgewählt ist und die andere Gruppe Wasserstoff bedeutet,
- n im Bereich von 2 bis 100 liegt,
- m 2 oder 3 bedeutet und daher die Länge der Polyalkylenoxidkette variieren kann,
- p 2 oder 3 bedeutet und
- G ausgewählt ist unter: Wasserstoff; einer $C_{1-6}$-Alkylgruppe, einer Gruppe -$(C_{m'}H_{2m'}O)_{n'}H$, worin n' im Bereich von 2 bis 100 liegt und m' 2 oder 3 bedeutet und daher die Länge der Kette variieren kann; und einem Kation,

mit der Maßgabe, daß mindestens einer der Parameter m und m' 2 bedeutet, wenn G eine Gruppe -$(C_{m'}H_{2m'}O)_{n'}H$ ist.

**3.** Verwendung nach einem der vorhergehenden Ansprüche, wobei G ein Gruppe $-(C_{m'}H_{2m'}O)_{n'}H$ bedeutet und n = n' und m = m'.

**4.** Verwendung nach einem der Ansprüche 1 bis 2, wobei G unter den Ionen von anorganischen oder organischen Basen, beispielsweise einem Alkalimetallion, insbesondere $Na^+$ oder $K^-$ oder einem quaternären Ammoniumion, und basischen Salzen von Lysin oder Arginin ausgewählt ist.

**5.** Verwendung nach einem der Ansprüche 1 bis 2, worin G Wasserstoff bedeutet.

**6.** Verwendung nach einem der vorhergehenden Ansprüche, wobei n im Bereich von 5 bis 60 und vorzugsweise im Bereich von 10 bis 30 liegt.

**7.** Verwendung nach einem der vorhergehenden Ansprüche, wobei die Gruppe R oder R', die von H verschieden ist, unter den geradkettigen Alkyl-1,2-en-Gruppen mit 8 bis 22 Kohlenstoffen ausgewählt ist.

**8.** Verwendung nach einem der Ansprüche 1 bis 2, wobei das Bernsteinsäueanhydrid-Derivat unten den Verbindungen einer der folgenden Formeln ausgewählt ist:

$$HOOC\text{-}(HR)C\text{-}CH_2\text{-}COO\text{-}(C_2H_4O)_{18}\text{-}H$$
mit R = n-Dodecyl-trans-2-en
$$HOOC\text{-}(HR)C\text{-}CH_2\text{-}COO\text{-}(C_2H_4O)_{18}\text{-}H$$
mit R = n-Hexadecyl-trans-2-en, und
$$HOOC\text{-}(HR)C\text{-}CH_2\text{-}COO\text{-}(C_2H_4O)_{18}\text{-}C_pH_{2p}\text{-}O\text{-}CO\text{-}CH_2\text{-}C(HR)\text{-}COOH$$
mit R = n-Hexadecyl-trans-2-en.

**9.** Verwendung nach einem der vorhergehenden Ansprüche, wobei die Zusammensetzung in Form einer Öl-in-Wasser-Emulsion, einer Wasser-in-Öl-Emulsion, einer multiplen Emulsion, einer wäßrigen Lösung, einer gelierten wäßrigen Lösung, einer Lotion, einer zweiphasigen Lotion, einer wäßrigen Lotion, einer wäßrig-alkoholischen Lotion, einer Creme, einer Milch oder eines Schaums vorliegt.

**10.** Verwendung nach einem der vorhergehenden Ansprüche, wobei die Zusammensetzung ferner mindestens einen grenzflächenaktiven Stoff und/oder mindestens ein Öl zum Abschminken enthält.

**11.** Verwendung nach Anspruch 10, wobei das Öl zum Abschminken unter den Estern ausgewählt ist, die aus einem geradkettigen oder verzweigten Alkohol mit 1 bis 17 Kohlenstoffatomen und einer geradkettigen oder verzweigten Fettsäure mit 3 bis 18 Kohlenstoffatomen hergestellt sind.

**12.** Verwendung nach einem der vorhergehenden Ansprüche, wobei die Zusammensetzung einen pH-Wert im Bereich von 5 bis 8 und vorzugsweise von 5,5 bis 7,5 aufweist.

**13.** Verwendung nach einem der vorhergehenden Ansprüche, wobei die Zusammensetzung eine Milch oder eine Lotion zum Abschminken der Augen und/oder der Haut, insbesondere der Gesichtshaut, ein Haarwaschmittel und/oder ein Duschgel ist.

**14.** Verfahren zur Reinigung und/oder zum Abschminken eines Trägers, wie beispielsweise der Haut, der Schleimhäute, der Semi-Schleimhäute, der Keratinsubstanzen und/oder der Augen, dadurch gekennzeichnet, daß auf den Träger eine Zusammensetzung aufgetragen wird, die mindestens ein Bernsteinsäureanhydrid-Derivat einer der folgenden Formeln I und II enthält:

$$GOOC\text{-}(HR)C\text{-}C(HR')\text{-}COO\text{-}(C_mH_{2m}O)_n\text{-}H \tag{I}$$

$$GOOC\text{-}(HR)C\text{-}C(HR')\text{-}COO\text{-}(C_mH_{2m}O)_n\text{-}C_pH_{2p}\text{-}O\text{-}CO\text{-}(HR')C\text{-}C(HR)\text{-}COOG \tag{II},$$

worin

- eine der Gruppen R1 und R' unter den geradkettigen oder verzweigten Alkyl- und Alkenylgruppen mit 6 bis 22 Kohlenstoffatomen ausgewählt ist und die andere Gruppe Wasserstoff bedeutet,
- n im Bereich von 2 bis 100 liegt,

- m 2 oder 3 bedeutet und daher die Länge der Polyalkylenoxidkette variieren kann,
- p 2 oder 3 bedeutet und
- G ausgewählt ist unter: Wasserstoff; einer $C_{1-6}$-Alkylgruppe, einer Gruppe $-(C_{m'}H_{2m'}O)_{n'}H$, worin n' im Bereich von 2 bis 100 liegt und m' 2 oder 3 bedeutet und daher die Länge der Kette variieren kann; und einem Kation,

mit der Maßgabe, daß mindestens einer der Parameter m und m' 2 bedeutet, wenn G eine Gruppe $-(C_{m'}H_{2m'}O)_{n'}H$ ist.